(19) Europäisches Patentamt
European Patent Office
Office européen des brevets

(11) Numéro de publication: **0 057 141 B1**

(12) **FASCICULE DE BREVET EUROPEEN**

(45) Date de publication du fascicule du brevet: **04.04.84**

(51) Int. Cl.³: **C 07 C 69/65**, C 07 C 67/14, A 61 K 31/235

(21) Numéro de dépôt: **82400116.8**

(22) Date de dépôt: **21.01.82**

(54) **Esters d'halogéno biphénylcarboxylates, leur procédé de préparation et leur application en tant que médicaments.**

(30) Priorité: **23.01.81 FR 8101424**

(43) Date de publication de la demande: **04.08.82 Bulletin 82/31**

(45) Mention de la délivrance du brevet: **04.04.84 Bulletin 84/14**

(84) Etats contractants désignés: **AT BE CH DE GB IT LI LU NL SE**

(56) Documents cités: **Aucun**

(73) Titulaire: **PIERRE FABRE S.A., 125, rue de la Faisanderie, F-75116 Paris (FR)**

(72) Inventeur: **Cousse, Henri, La Foun de los Nobios Chemin de Lastinos, F-81100 Castres (FR)**
Inventeur: **Mouzin, Gilbert, 21, rue Sainte-Foy, F-81100 Castres (FR)**
Inventeur: **Rieu, Jean-Pierre, La Vixère-Haute Avenue du Sidobre, F-81100 Castres (FR)**
Inventeur: **Delhon, André, 36, rue Hector Berlioz, F-81100 Castres (FR)**

(74) Mandataire: **Doat, Jean-Pierre, PIERRE FABRE S.A. Service Propriété Industrielle 17, Avenue Jean Moulin, F-81106 Castres Cédex (FR)**

**EP 0 057 141 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Esters d'halogéno biphénylcarboxylates, leur procédé de préparation et leur application en tant que médicaments

La présente invention, réalisée au Centre de Recherches Pierre FABRE, a pour objet de nouveaux esters d'acides halogéno biphénylcarboxyliques, leur procédé de préparation et leur application en tant que médicaments. Ils sont utiles notamment dans le traitement des hyperlipidémies. L'invention vise également les compositions pharmaceutiques contenant ces principes actifs.

Les composés chimiques selon la présente invention répondent à la formule générale I:

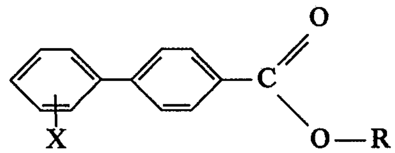

(I)

dans laquelle:

X représente un halogène en position 2' et 3'.

R représente un alcoyle linéaire ou ramifié contenant de 1 à 16 atomes de carbone, un cycloalcoyle, un benzyle, un alcoylamino, un alcoylpyridyle. L'invention s'applique également aux sels des composés de formule (I) quand R représente un groupe alcoylamino ou alcoyl pyridyle, avec des acides thérapeutiquement acceptables.

L'invention concerne également un procédé de préparation des composés de formule (I), caractérisé en ce que l'on condense un chlorure d'acide de formule générale II:

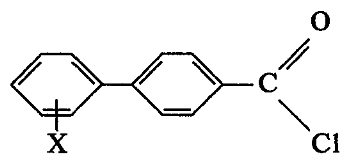

(II)

avec un alcool de formule générale III: H – O – R.

X et R ont la signification donnée à propos de la formule générale (I). Les chlorures d'acides intermédiaires de formule générale (II) sont préparés par action du chlorure de thionyle sur les acides de formule générale IV:

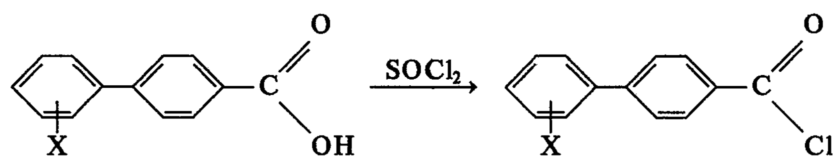


(IV)

Les acides biphényls carboxyliques de formule IV sont préparés selon:

a) W.S.M. GRIEVE et D.H. HEY, J. Chem. Soc., 1933, 968.
b) E.E. BERLINER et E.A. BLOMMERS, J. Amer. Chem. Soc., 1951, 73, 2479.
c) D.J. BYRON, G.W. GRAY et R.C. Wilson, J. Chem. Soc., 1966, 840.

La présente invention sera décrite ci-après plus en détails à propos des exemples non limitatifs suivants:

### A) Synthese

#### Exemple 1

#### Préparation du chloro-2' biphényl-4 carboxylate d'éthyle

##### a) Préparation du chlorure d'(orthochlorophényl)-4 benzoyle

Dans un ballon de 500 ml renfermant 50 g (0,214 mole) d'acide (ortho chloro phényl)-4 benzoïque, on ajoute goutte à goutte 163,1 g (1,37 mole) de chlorure de thionyle en agitant mécaniquement et en chauffant le mélange à 120° C.

Le reflux est maintenu pendant 4 heures. L'excès de chlorure de thionyle est distillé sous pression rédiute, puis on reprend le résidu dans du toluène et évapore jusqu'à siccité.

On renouvelle encore une fois cette dernière opération puis l'huile résiduelle est rectifiée sous vide. On obtient 40 g de produit (rendement 73%). $Eb_{0,01}$: 137 – 140° C.

Spectre IR: $\nu cm^{-1} = 1780$ et 1740 (chlorure d'acide).

Spectre RMN: ($CDCl_3$/TMS) $\delta$ppm: 7,45 (m, 6H, aromatique); 8,1 (d, 2H aromatiques ortho du C=O).

b) Préparation du chloro-2′ biphényl-3 carboxylate d'éthyle

Dans un tricol de 100 ml, une solution de 40mmoles de chlorure (d'ortho chlorophényl)-4 benzoyle dans 10 ml de diméthyl acétamide est ajoutée goutte à goutte en 20 minutes sous agitation à une solution de 42 mmoles d'alcool éthylique dans 20 ml de pyridine.

L'introduction du chlorure d'acide est réalisée de telle sorte que la température réactionnelle soit toujours voisine de 20° C.

Après 1 heure d'agitation, le mélange réactionnel est jeté dans de l'eau glacée. Extraire 2 fois à l'éther éthylique. La phase organique est lavée au bicarbonate, puis à l'eau jusqu'à neutralité. Sécher sur sulfate de sodium, après filtration et évaporation du solvant, l'huile résiduelle est dissoute dans l'éthanol à 65° GL à chaud, glacer et filtrer. On recupère avec un rendement de 82% le produit de formule:

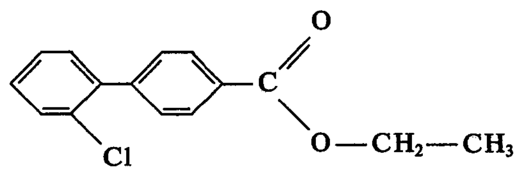

Formule brute: $C_{15}H_{13}ClO_2$
Masse moléculaire: 260,7.
Cristaux: blancs.
Point de fusion: 41° C.
Chromatographie sur plaque:

- support: gel de silice 60 F 254 Merck
- solvant: acétate d'éthyle — éther de pétrole 10/90
- révélation: UV et iode.
- Rf: 0,6.

Solubilités: insoluble dans l'eau. Soluble à 1% dans le propylène glycol, à 20% dans le diméthyl sulfoxyde et à 5% dans l'éthanol.

Exemple 2

Préparation du chloro-2′ biphényl-4 carboxylate d'isopropyle

D'une façon similaire à celle décrite dans l'exemple 1b, mais en utilisant l'alcool isopropylique, on obtient le produit de formule:

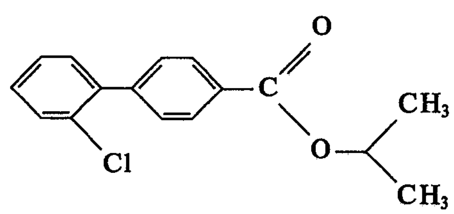

Formule brute: $C_{16}H_{15}ClO_2$.
Masse moléculaire: 274,7.
Huile incolore: $Eb_{0,02} = 91°$ C – $n_D^{24} = 1,5745$
Chromatographie sur plaque:

- support: gel de silice 60 F 254 Merck
- solvant: acétate d'éthyle — éther de pétrole 10/90

— révélation: UV et iode.
— Rf: 0,55.

Solubilities: insoluble dans l'eau. Soluble à 1% dans le propylène glycols, à 10% dans le DMSO et à 2% dans l'éthanol.

Exemple 3

Préparation du chloro-2' biphényl-4 carboxylate de n-octyle

D'une façon similaire à celle décrite dans l'exemple 1b, mais en utilisant l'alcool octylique, on obtient le produit de formule:

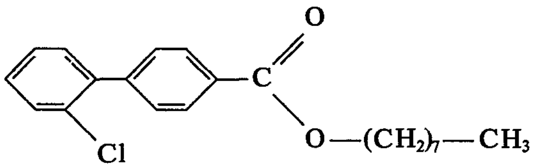

Formule brute: $C_{21}H_{25}ClO_2$
Masse moléculaire: 344,8.
Huile incolore — $Eb_{0,02} = 156-158°C$ — $n_D^{22} = 1,5510$.
Chromatographie sur plaque:

— support: gel de silice 60 F 254 Merck
— solvant: acétate d'éthyle — éther de pétrole 10/90
— révélation: UV et iode
— Rf: 0,65.

Solubilités: insoluble dans l'eau. Soluble à 0,1% dans le propylène glycol, à 20% dans le DMSO et à 5% dans l'éthanol.

Exemple 4

Préparation du chloro-2' biphényl-4 carboxylate de n-hexadecyle

D'une façon similaire à celle décrite dans l'exemple 1b, mais en utilisant l'hexadécanol, on obtient le produit de formule:

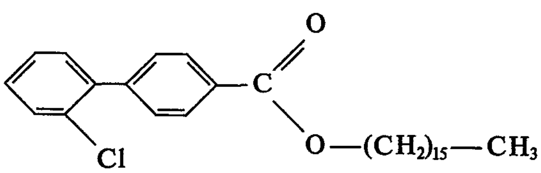

Formule brute: $C_{29}H_{41}ClO_2$.
Masse moléculaire: 457,1.
Cristaux: blancs.
Point de fusion: 34 à 35° C
Chromatographie sur plaque:

— support: gel de silice 60 F 254 Merck
— solvant: acétate d'éthyle — hexane 5/95
— révélation: UV et iode.
— Rf: 0,62.

Solubilités: insoluble dans l'eau. Soluble à 0,1% dans le propylène glycol et dans le DMSO, à 0,5% dans l'éthanol et 1% dans le DMA.

Exemple 5

Préparation du chloro-2' biphényl-4 carboxylate de triméthyl-3-3-5 cyclohexyle

D'une façon similaire à celle décrite dans l'exemple 1b, mais en utilisant le dihydro isophorol, on obtient le produit de formule:

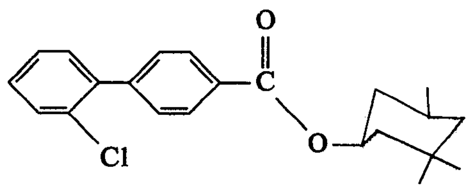

Formule brute: $C_{22}H_{25}ClO_2$
Masse moléculaire: 356,9.
Cristaux: blancs.
Point de fusion: 66° C.
Chromatographie sur plaque:

- support: gel de silice 60 F 254 Merck
- solvant: acétate d'éthyle — éther de pétrole 10/90
- révélation: UV et iode
- Rf: 0,63.

Solubilités: insoluble dans l'eau. Soluble à 0,1% dans le propylène glycol, à 20% dans le DMSO et à 2% dans l'éthanol.

Exemple 6

Préparation du chloro-2' biphényl-4 carboxylate de benzyle

D'une façon similaire à celle décrite dans l'exemple 1b, mais en utilisant l'alcool benzylique, on obtient le produit de formule:

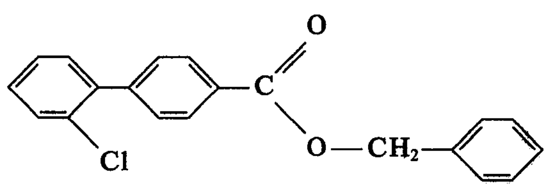

Formule brute: $C_{20}H_{15}ClO_2$
Masse moléculaire: 322,8.
Cristaux blancs.
Point de fusion: 44° C.
Chromatographie sur plaque:

- support: gel de silice 60 F 254 Merck
- solvant: Ether de pétrole — acétate d'éthyle 90/10
- révélation: UV et iode
- Rf: 0,50.

Solubilités: Insoluble dans l'eau. Soluble à 0,2% dans le propylène glycol, à 20% dans le DMSO et à 2% dans l'éthanol.

Exemple 7

Préparation du bromo-3' biphényl-4 carboxylate d'isopropyle

D'une façon similaire à celle décrite dans l'exemple la et 1b, mais en utilisant le chlorure de (métabromo phényl)-4 benzoyle et l'alcool isopropylique, on obtient le produit de formule:

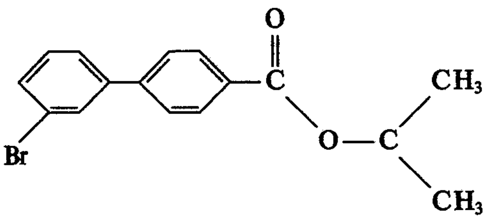

Exemple 8

Préparation du fluoro-2′ biphényl-4 carboxylate d'éthyle

D'une façon similaire à celle décrite dans l'exemple 1a et 1b, mais en utilisant le chlorure d'(ortho fluoro phényl)-4 benzoyle on obtient le produit de formule:

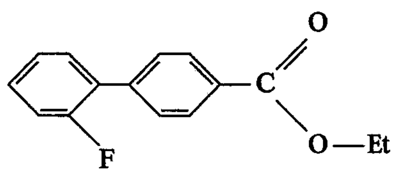

Exemple 9

Préparation du chloro-2′ biphényl-4 carboxylate de diméthylamino-2 éthyle, chlorhydrate

Dans un tricol de 100 ml refroidi dans un bain d'eau froide, une solution de 10,04 g (40 mmoles) de chlorure d'(orthochloro phényl)-4 benzoyle dans 10 ml de diméthyl acétamide est ajoutée sous forte agitation à un mélange de 4,22 ml (3,74 g) 42 mmoles de diméthylamino éthanol dans 25 ml de pyridine.

L'addition est réalisée de telle sorte que la température de la solution réactionnelle soit voisine de 20° C.

L'addition du chlorure d'acide dure 30 minutes. Le mélange réactionnel est ensuite jeté dans l'eau glacée, on sature avec une solution de carbonate de sodium et extrait 3 fois à l'éther éthylique.

La phase organique est lavée abondamment à l'eau et séchée sur sulfate de sodium.

Après évaporation de l'éther éthylique, la base résiduelle est reprise dans l'éther éthylique et chlorhydratée par addition d'une solution éthanolique d'acide chlorhydrique.

Les cristaux obtenus sont recristallisés dans un mélange méthyl éthyl cétone et isopropanol. On récupère avec un rendement de 65% le produit de formule:

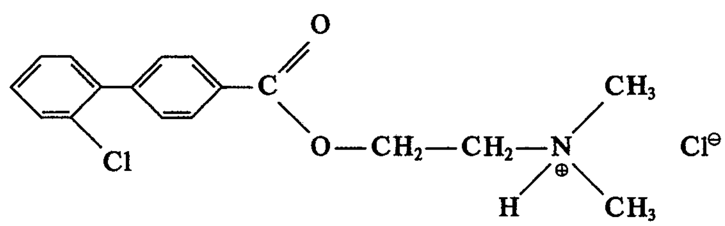

Formule brute: $C_{17}H_{19}Cl_2NO_2$.
Masse moléculaire: 340,2.
Cristaux: blancs.
Point de fusion: 164° C.
Chromatographie sur plaque:

- support: gel de silice 60 F 254 Merck
- solvant: chloroforme — méthanol 90/10
- révélation: UV et iode
- Rf: 0,39.

Solubilités: soluble dans l'eau à 10%, dans le DMSO à 12%.

Exemple 10

Préparation du chloro-2' biphényl-4 carboxylate de pyridyl-3 méthyle, chlorhydrate

D'une façon similaire à celle décrite dans l'exemple 9, mais en utilisant le 3-nicotinol, on obtient le produit de formule:

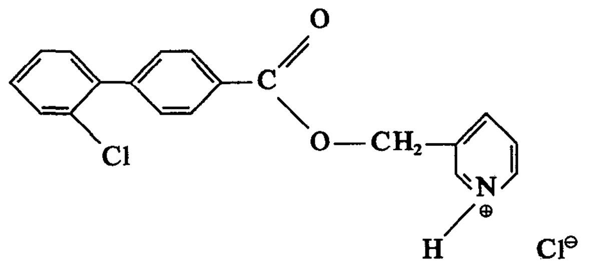

Formule brute: $C_{19}H_{15}Cl_2NO_2$.
Masse moléculaire: 360,2.
Cristaux blancs.
Point de fusion: 120° C.
Chromatographie sur plaque:

- support: gel de silice 60 F 254 Merck
- solvant: chloroforme — méthanol 90/10
- révélation: UV et iode.
- Rf: 0,79.

Solubilités: insoluble dans l'eau. Soluble à 0,1% dans le propylène glycol et à 12% dans le DMSO.

B) Pharmacologie

A titre d'exemple, on indiquera ci-après quelques résultats des divers essais toxicologiques et pharmacologiques effectués sur les composés de l'invention.

a) Etude de toxicité

Les composés de la présente invention ont été soumis à des contrôles de toxicité.

L'étude de la toxicité a été effectuée chez la souris conventionnelle pesant de 20 à 22 grammes.

Les substances ont été administrées par voie intraveineuse et orale. La $DL_{50}$ est calculée selon la méthode de MILLER et TAINTER (Proc. Soc. Exper. Biol. Med., 1944, 57, 261).

Par voie intraveineuse les $DL_{50}$ sont comprises entre 100 et 200 mg/kg. Par voie orale les $DL_{50}$ sont comprises entre 1000 mg et 2000 mg/kg.

b) Propriétés pharmacologiques

Ci-après sont rapportés les résultats obtenus avec les produits les plus intéressants (exemple N° 1, N° 2, N° 9); ils sont comparés au clofibrate.

Test portant sur 4 jours de traitement voie orale selon BUCHANAN, SPRANCMANIS et PARTYKA, J. Med. Chem. 12, 1969, 100.

| Produits | Dose orale mg/kg | % de baisse du cholestérol par rapport au témoin |
|---|---|---|
| Ex. N°1 | 10 | −20 |
| | 20 | −40 |
| | 100 | −90 |
| Ex. N°2 | 10 | −17 |
| | 20 | −30 |
| | 100 | −85 |
| Ex. N°9 | 10 | −15 |
| | 20 | −32 |
| | 100 | −87 |
| Clofibrate | 200 | −34 |

Compte-tenu de leurs propriétés pharmacologiques et de leur faible toxicité, les composés de l'invention et plus particulièrement le composé de l'exemple N° 1 peuvent être utilisés en thérapeutique dans le traitement des différents types d'hyperlipidémies en vue de la prévention et du traitement de l'athérosclérose.

**Revendications**

1. A titre de composés chimiques nouveaux, les esters d'halogéno biphényl carboxylates de formule générale (I):

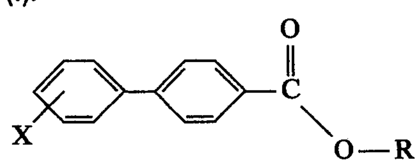

dans laquelles:
X représente un halogène en position 2′ ou 3′.
R représente un alcoyle linéaire ou ramifié contenant de 1 à 16 atomes de carbone, un cycloalcoyle, un benzyle, un alcoyl amino, un alcoyl pyridyle.

Ainsi que les sels avec les acides minéraux ou organiques thérapeutiquement acceptables, quand R représente un groupe alcoyl amino ou alcoyl pyridyle.

2. Les composés de formule générale I et plus particulièrement les dérivés chlorés en position 2.

3. Les composés de formule générale I caractérisés par le fait qu'ils sont choisis parmi:

— le chloro-2′ biphényl-4 carboxylate d'éthyle
— le chloro-2′ biphényl-4 carboxylate d'isopropyle
— le chloro-2′ biphényl-4 carboxylate de n-octyle
— le chloro-2′ biphényl-4 carboxylate de n-hexadecyle
— le chloro-2′ biphényl-4 carboxylate de triméthyl-3-3-5 cyclohexyle
— le chloro-2′ biphényl-4 carboxylate de benzyle
— le bromo-3′ biphényl-4 carboxylate d'isopropyle
— le fluoro-2′ biphényl-4 carboxylate d'éthyle
— le chloro-2′ biphényl-4 carboxylate de diméthylamino-2 éthyle, chlorhydrate
— le chloro-2′ biphényl-4 carboxylate de pyridyl-3 méthyle, chlorhydrate.

4. Procédé de préparation des composés de formule générale (I) selon les revendications 1 à 3, caractérisé par le fait que l'on condense un chlorure d'acide de formule (II):

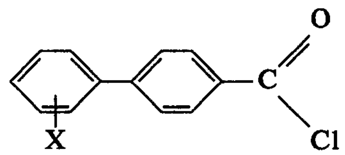

(II)

avec un alcool de formule (III):

H–O–R (III)

X et R ont la signification donnée à propos de la formule générale (I).

5. A titre de médicaments nouveaux utiles dans le traitement des troubles provoqués par l'athérosclérose les composés selon les revendications 1 à 3.

6. Les compositions pharmaceutiques qui facilitent la biodisponibilité contenant comme principe actif au moins un produit selon les revendications 1 à 3.

**Patentansprüche**

1. Als neue chemische Verbindungen, die Ester der Halogenbiphenylcarboxylate der allgemeinen Formel I:

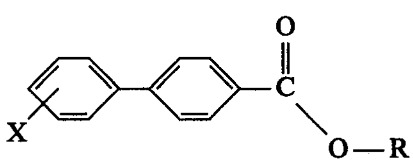

worin:
X ein Halogen in Position 2′ oder 3′ bedeutet,
R bedeutet einen geradkettigen oder verzweigten Alkylrest, der 1 bis 16 Kohlenstoffatome enthält, einen Cycloalkyl-, Benzyl-, Alkylamino, Alkylpyridylrest. Ebenso die Salze mit den therapeutisch akzeptablen Mineral- oder organischen Säuren, wenn R eine Alkylaminogruppe oder eine Alkylpyridylgruppe bedeutet.

2. Verbindungen der allgemeinen Formel I und insbesondere die Derivate, die in Position 2 chloriert sind.

3. Verbindungen der allgemeinen Formel I, dadurch gekennzeichnet, daß sie ausgewählt sind aus:

- Chlor-2′-biphenyl-4-carboxylat des Ethyls
- Chlor-2′-biphenyl-4-carboxylat des Isopropyls
- Chlor-2′-biphenyl-4-carboxylat n-Octyls
- Chlor-2′-biphenyl-4-carboxylat des n-Hexadecyls
- Chlor-2′-biphenyl-4-carboxylat des Trimethyl-3,3,5-cyclohexyls
- Chlor-2′-biphenyl-4-carboxylat des Benzyls
- Brom-3′-biphenyl-4-carboxylat des Isopropyls
- Fluor-2′-biphenyl-4-carboxylat des Ethyls
- Chlor-2′-biphenyl-4-carboxylat des Dimethylamino-2-ethylchlorhydrats
- Chlor-2′-biphenyl-4-carboxylat des Pyridyl-3-methyl-chlorhydrats

4. Verfahren zur Herstellung der Verbindungen der allgemeinen Formel I nach einem der Ansprüche 1 bis 3, dadurch gekennzeichnet, daß man ein Säurechlorid der Formel II:

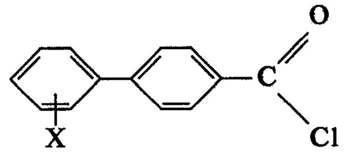

(II)

kondensiert mit einem Alkohol der Formel III:

H–O–R (III)

X und R haben die Bedeutung, wie in der allgemeinen Formel I angegeben.

5. Verwendung der Verbindungen nach einem der Ansprüche 1 bis 3 als neue Medikamente zur Behandlung der Leiden, die durch die Arteriosklerose hervorgerufen werden.

6. Pharmazeutische Zusammensetzungen, die die biologische Verfügbarkeit erleichtern, enthaltend als aktiven Bestandteil mindestens ein Produkt nach einem der Ansprüche 1 bis 3.

**Claims**

1. As novel chemical compounds, the halobiphenylcarboxylate esters of the general formula (I)

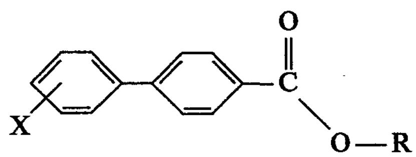

in which
X represents a halogen in the 2'- or 3'-position,
R represents a straight-chain or branched alkyl containing from 1 to 16 carbon atoms, a cycloalkyl, a benzyl, an aminoalkyl or a pyridylalkyl,
and the salts with therapeutically acceptable mineral or organic acids when R represents an aminoalkyl group or a pyridylalkyl group.

2. The compounds of the general formula I and, more especially, the derivatives chlorinated in the 2-position.

3. The compounds of the general formula I, characterised by the fact that they are selected from:

ethyl 2'-chloro-4-biphenylcarboxylate
isopropyl 2'-chloro-4-biphenylcarboxylate
n-octyl 2'-chloro-4-biphenylcarboxylate
n-hexadecyl 2'-chloro-4-biphenylcarboxylate
3,3,5-trimethylcyclohexyl 2'-chloro-4-biphenylcarboxylate
benzyl 2'-chloro-4-biphenylcarboxylate
isopropyl 3'-bromo-4-biphenylcarboxylate
ethyl 2'-fluoro-4-biphenylcarboxylate
2-dimethylaminoethyl 2'-chloro-4-biphenylcarboxylate hydrochloride
3-pyridylmethyl 2'-chloro-4-biphenylcarboxylate hydrochloride.

4. Process for the preparation of the compounds of the general formula (I) according to claims 1 to 3, characterised by the fact that an acid chloride of the formula (II)

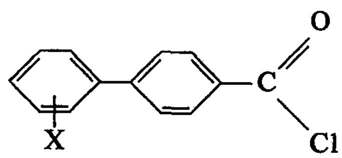

(II)

is condensed with an alcohol of the formula (III)

$$H-O-R \qquad (III)$$

in which X and R have the meanings given with regard to the general formula (I).

5. The compounds according to claims 1 to 3 as novel medicaments that can be used in the treatment of disorders caused by arteriosclerosis.

6. Pharmaceutical compositions that facilitate bioavailability containing as active ingredient at least one product according to claims 1 to 3.